# EUROPEAN PATENT APPLICATION

(11) **EP 4 703 506 A1**
(43) Date of publication of application: **04.03.2026**
(21) Application number: 24196974.0
(22) Date of filing: 28.08.2024
(51) Int. Cl.: D04H 1/425, D04H 1/541, D04H 1/544, D04H 3/007, A61F 13/511, D01F 6/06, D04H 1/4291

(54) **ABSORBENT ARTICLE WITH A DURABLY HYDROPHILIC CARDED NONWOVEN**

(71) Applicant: The Procter & Gamble Company, Cincinnati, OH 45202 (US)
(72) Inventor: LINDNER, Torsten, 65824 Schwalbach am Taunus (DE); ERDEM, Gueltekin, Cincinnati, 45202 (US); GUTMANN, Patrick, 86399 Bobingen (DE); BERGMANN, Mads Rudbeck, 6800 Varde (DK)
(74) Representative: P&G Patent Germany

(57) **Abstract**

An absorbent article comprising a durably hydrophobic carded nonwoven (110) in particular as topsheet or acquisition layer. The nonwoven comprises synthetic staple fibers (120) that comprise or consist of a hydrophilic polypropylene-based composition. The hydrophilic polypropylene-based composition comprising a polypropylene-based matrix, an ethylene-propylene copolymer additive and a hydrophilic melt additive.

## Description

### FIELD OF THE INVENTION

The invention relates to an absorbent article such as a diaper comprising a carded nonwoven comprising staple propylene-based synthetic fibers having durably hydrophilic properties.

### BACKGROUND OF THE INVENTION

Absorbent articles for personal hygiene such as diapers for babies or incontinent adults are designed to absorb and contain body exudates, in particular urine. These absorbent articles typically comprise several layers, in particular a fluid permeable topsheet on the body-facing side, a fluid impermeable backsheet on the garment-facing side and an absorbent core sandwiched in-between these layers. An acquisition layer is typically disposed underneath the topsheet. Pant-shaped products also typically comprise an inner and outer nonwoven belt.

Nonwovens are commonly used in disposable absorbent articles. Nonwovens are affordable, safe and versatile. They can display a variety of properties including softness, liquid barrier properties or alternatively liquid acquisition properties, depending on their structure and any post-treatments. A topsheet may be typically formed by a soft nonwoven which is easily wettable. A backsheet is typically a laminate of a liquid barrier plastic film and a nonwoven outer cover having a soft feel.

Nonwovens are sheet or web structures bonded together by entangling fibers or filaments mechanically, thermally, or chemically. They are flat, porous sheets that are made directly from separate fibers. They are not made by weaving or knitting and do not require converting the fibers to yarn. Common processes to make nonwovens are meltblowing, spunbonding, solvent spinning, electrospinning, carding and airlaying. The basis weight of nonwoven webs is usually expressed in grams per square meter (g/m² or gsm).

Spunlaid nonwovens are made in one continuous process where molten polymer granules are extruded into filaments through so called spinnerets. The continuous filaments are stretched and quenched before being deposited on a conveyor belt to form a uniform web. Spunlaid nonwovens have an increased strength compared to carded nonwovens, due to the attenuation of the filaments. The downside is that the choice of raw materials is more restricted. The term "spunbonded nonwoven" designates thermo bonded spunlaid.

Carded nonwovens on the other hand are made from staple fibers that are mechanically processed. Staple fibers are fibers having a discrete length which is much shorter than spunlaid fibers. In a typical carding process, bales of staple fibers are brought to a carding line where the bales are unwrapped. The bales are opened at a bale opener and the fibers are initially separated and transported by conveyer belt to the fiber preparation step where further fiber opening is taking place. The staple fibers then enter a master chute where the web going into the card is prepared. The master chute is also controlling the dosing of the fibers and ensures that there is an even distribution of fibers throughout the width of the web. Inside the card, the fibers are distributed in both the machine direction, MD, and cross direction, CD, depending on several parameters such as line speed, fiber cohesion and randomizers amongst other. The carded web obtained has limited initial strength and is therefore typically further consolidated. Known consolidation methods are calendaring, air-through bonding, resin bonding and needle-punching.

Carded nonwovens have several advantages over spunbonded nonwovens. Carded nonwovens provide material design flexibility to deliver different benefits. For instance, it is possible to include natural fibers such as cotton fibers or other thermally un-bondable natural fibers (e.g., hemp) into the staple fiber blends. Carded nonwovens can also have non-uniform or layered structures which may be desired in certain applications.

It is desirable for certain applications such as topsheet or acquisition layer that the nonwoven be liquid permeable and hydrophilic. Synthetic fibers are however inherently hydrophobic. Typically, a hydrophilic surfactant is added by spraying on the surface of the nonwoven or passing the nonwoven through a surfactant solution to render it hydrophilic. It has also been suggested to add hydrophilic melt additives in the melt from which the synthetic fibers are formed. However, these surfactants are often washed-off after a first gush of e.g. urine, reducing the wettability of the fibers during use. Accordingly, there is a need for a nonwoven having the benefits of carded nonwoven while also being durably hydrophilic.

### SUMMARY OF THE INVENTION

The present invention is in a first aspect for an absorbent article comprising a carded nonwoven comprising synthetic staple fibers. The synthetic staple fibers comprise or consist of a hydrophilic polypropylene-based composition, said hydrophilic polypropylene-based composition comprising a polypropylene-based matrix, an ethylene-propylene copolymer additive and a hydrophilic melt additive. The nonwoven may be consolidated by a method selected from calendering, air-through bonding, resin bonding or needle-punching. The carded nonwoven is durably hydrophilic, that is it remains hydrophilic even after exposure to an aqueous fluid.

Further advantageous features of the invention are listed in the dependent claims herein.

### BRIEF DESCRIPTION OF THE DRAWINGS

Fig. 1A,B show SEM pictures of the calendered side of an exemplary nonwoven according to the invention at different magnifications;
Fig. 2A,B show SEM pictures of the cross-section of the same nonwoven;
Fig. 3 shows the wearer-facing side of an exemplary taped diaper with some layers partially removed to expose the inner layers;
Fig. 4 is a cross-section of the diaper of Fig. 3.

### DETAILED DESCRIPTION OF THE INVENTION

### Carded nonwovens and synthetic staple fibers

The absorbent article of the present invention comprises a carded nonwoven comprising staple synthetic fibers. The synthetic staple fibers comprise or consist of a hydrophilic polypropylene-based composition, the composition comprising a polypropylene-based matrix, an ethylene-propylene copolymer additive and a hydrophilic melt additive, as further exemplified and detailed below.

These synthetic staple fibers may be a) mono-component fibers consisting of the hydrophilic polypropylene-based composition, or b) muti-component fibers, in particular bi-component fibers, wherein one of the components is the hydrophilic polypropylene-based composition, or a mixture of fibers according to a) and b). The bi-component fibers may in particular have a sheath/core structure, wherein the sheath consists of the hydrophilic polypropylene-based composition. The core may be a PP based polymer or other suitable polymer having a higher melting point than the polymer of the sheath structure, such as PET, as is known in the art.

The synthetic staple fibers of the invention can be obtained by melting the polypropylene-based matrix and adding the ethylene-propylene copolymer and hydrophilic melt additives to this melt, as well as other additives if desired. The molten mixture is then extruded in the form of continuous fibers, which are stretched and cut to make staple fibers of a desired linear density and length. The PP-based matrix resin and the additives are typically provided as pellets by their respective suppliers, as is known in the art. The linear density for the synthetic fibers may be for example in the range of from 1.5 to 2.5 dtex. The fiber diameter of the synthetic staple fibers may be for example in the range of 14 µm to 20 µm.

The synthetic staple fibers are carded as is known in the art. Natural fibers may be mixed with the synthetic staple fibers if desired before the fibers are carded. Carding already provides enough integrity to form a nonwoven. Subsequent consolidation of the carded staple fibers is typically required as is known in the art to make a consolidated carded nonwoven. Consolidation methods of the carded fibers include in particular one of calendering, air-through bonding, resin bonding and needle-punching.

Calendering is a thermal bonding method that uses heat and pressure applied between two heated rollers to locally weld the staple fibers together at high speed. One roller may be smooth, and the other roller may be engraved with a pattern, providing the desired bonding characteristics for the final nonwoven. The two rollers are pressed together and the combination of pressure and heat results in thermal bonding points where the two rollers are touching. These bonding points in the final nonwoven are what gives the tensile properties, for both machine direction (MD) and cross direction (CD).

Calendering involves locally fusing the synthetic fibers by a plurality of bond points. The bonding area is the area of the nonwoven occupied by the calendering bond points. The bond points can have shapes used in the art, for example rectangular or diamond-shaped, and are typically oval shaped. Typically, the bonding area may range from 8% to 30% of the surface of the nonwoven. Exemplary values for a calendered bonding pattern include a bonding area in the range of 10% to 16%, a bonding density (number of bond points per area) in the range of about 50/cm² to about 60/cm² and a pitch (distance form center to center of the bond points) is in the range of 1.40 mm to 2.20 mm in both cross direction and machine direction.

Air-through bonding (ATB) is another type of thermal bonding, and is typically used when the staple fibers comprise bicomponent fibers. Bicomponent fibers typically have a core/sheath structure, with the sheath component having a lower melting point than the core component. For example, bicomponent fibers may comprise Polyethylene (PE) in the sheath and Polypropylene (PP) or Polyethylene terephthalate (PET) in the core. PE has a lower melting temperature than PP or PET, and junction points can thus be created by fusing the PE sheath of the bicomponent fibers to other fibers, including PE mono-fibers or the PE sheath of other bicomponent fibers. A hot air stream is used to melt the sheath of the bicomponent fibers.

Resin bonding and needle-punching are alternative consolidating methods known in the art.

After the consolidation step, the resulting carded consolidated nonwoven can be wound up on large rolls for storage and transport.

### PP-based matrix

Polypropylene (PP) is a thermoplastic polymer that is widely used in various industries due to its versatile properties. Polypropylene is known for its high strength, good chemical resistance, low density, and availability. Polypropylene has a relatively low density, making it lightweight compared to many other plastics. The density of PP typically ranges from 0.895 to 0.92 grams per cubic centimeter (g/cm³). The synthetic fibers of the invention comprise a PP-based matrix as main component. The melting point of polypropylene typically ranges from 130 °C to 180 °C. This property allows PP to be easily melted and molded into various shapes. The PP-based matrix useful for the present invention include PP Homopolymers ("PP-H") and PP Copolymers ("CoPP").

Polypropylene Homopolymer is the most basic form of polypropylene and consists of only propylene monomers. It offers high stiffness, strength, and good chemical resistance. In terms of tacticity, polypropylene (PP) homopolymers can be classified into three main types: Isotactic Polypropylene (i-PP), Syndiotactic Polypropylene (s-PP) and Atactic Polypropylene (a-PP).

Isotactic polypropylene is the most common and commercially available form of PP. In isotactic PP, all the methyl groups (CH3) in the polymer chain are positioned on the same side, resulting in a regular and orderly arrangement of side groups. This arrangement contributes to a high degree of crystallinity, which enhances the polymer's mechanical strength, stiffness, and heat resistance.

In syndiotactic polypropylene, the methyl groups alternate in a regular pattern along the polymer chain. The syndiotactic structure leads to a lower degree of crystallinity and decreased polymer chain mobility, resulting in improved transparency and increased flexibility compared to isotactic PP.

Atactic polypropylene has a random arrangement of side groups along the polymer chain. This results in a highly amorphous structure with low crystallinity and poor mechanical properties. Atactic PP is usually produced as a byproduct during PP synthesis and is less commonly used in industrial applications.

The tacticity of polypropylene significantly affects its physical and mechanical properties, such as crystallinity, melting behavior, thermal stability, and flexibility. The specific tacticity of PP can be controlled during the polymerization process by using specific catalysts and reaction conditions.

The PP polymer matrix of the invention is preferably a PP Homopolymer (PP-H), however a CoPP may also be considered for the PP-based matrix. In that case, the CoPP and the ethylene-propylene copolymer additives are different components of the synthetic fibers. While suitable ethylene-propylene copolymer additives have substantially amorphous character with an enthalpy of crystallization preferably in the range of 0.5 J/g to 40 J/g, suitable PP polymer matrix (PP-H or CoPP) have much higher enthalpy of crystallization, typically exceeding 40 J/g, or 45 J/G, or 50 J/g, e.g. of from 50 J/g to 200 J/g. For the same reasons, the PP-based matrix has typically a melting point which is much higher (at least 20 °C or at least 40 °C difference) than the melting point of the ethylene-propylene copolymer additive.

PP copolymers ("CoPP") comprise at least 50% of propylene monomers as main component, by weight, and one or more other monomers, in particular ethylene. CoPP include random copolymers and block copolymers. Random PP copolymers contain a small amount of comonomer (usually ethylene) randomly distributed within the polymer chain. It exhibits improved clarity, impact resistance, and flexibility compared to homopolymer polypropylene.

Block PP Copolymer are produced by polymerizing propylene and a different comonomer such as ethylene in separate polymerization steps. It combines the properties of both homopolymer and random copolymer polypropylene, offering a balance between stiffness, impact resistance, and transparency.

The Melt Flow Index of suitable PP resins that may be used as PP-based matrix may typically be in the range of 4 g/10 min to 40 g/10 min (MFI being measured at 230°C*2.16 kg - ISO 1133, procedure A), in order to ease fiber extrusion while providing sufficient resilience to the PP-based matrix. Preferred range of the MFI for the PP-based matrix is in the range of from 5 g/10 min to 30 g/10 min, and more preferred range is of from 6 g/10 min to 20 g/10 min. This grade enables on the one hand particularly good staple fiber spinning behavior and at the same time optimal thermal bonding performance.

An exemplary PP-H is Total PPH 7059, a PP homopolymer with a melt flow index of about 13 g/10 min, which was used in the example below as the resin for making the synthetic PP-based staple fibers. Another PP-H commercial example is PP HSP165G (ex. Braskem), which has a Melting Peak Temperature (Tmp) of 166.5 °C as measured by the Melting Peak Temperature Test Method disclosed herein.

An exemplary CoPP that may be used as PP-based matrix of the invention is a metallocene random copolymer sold by Total under the Tradename Lumicene^{®} MR10YN9, having a Melt Flow Index of about 10 g/10 min, and a Melting Peak Temperature (Tmp) of 137.8 °C as measured by the Melting Peak Temperature Test Method disclosed herein.

### Ethylene-propylene copolymer additive(s)

The hydrophilic PP-based composition comprises, in addition to the PP polymer matrix, at least one ethylene-propylene copolymer as additive. Suitable ethylene-propylene copolymer additives are formed from olefin monomer units, i.e., propylene with ethylene copolymerized together. The polymers are preferably metallocene-technology based, and thus are produced using metallocene catalysts, but other catalysts such as Ziegler Natta can also be used. Metallocene-technology based polymers typically have a regular spatial repeat monomer unit distribution and a narrow molecular weight distribution, as is known in the art.

The ethylene-propylene copolymers may typically comprise at least 50% by weight of propylene units, in particular at least 60%, or at least 70%, or at least 80% by weight. The remaining monomers are ethylene monomers, and optionally other alpha olefin monomers that may be present in the co-polymers, for example 4-methyl-1-pentene, pentene-1, 2-methylpentene-1, 3-methylbutene-1, heptene-1, dimethylpentene-1, trimethylbutene-1, ethylpentene-1, methylpentene-1, trimethylpentene-1, methylethylpentene-1, 1-octene, diethylbutene-1, propylpentane-1, decene-1, methylnonene-1, nonene-1, trimethylheptene-1, methylethylbutene-1, dodecene-1, and hexadodecene-1, and combinations thereof. The exact monomer distribution is typically published by the copolymer material supplier, but can also be determined by a suitable method, such as nuclear magnetic resonance or infrared spectroscopies.

The ethylene-propylene copolymer additive is preferably comprised of a single material as defined above, as this simplifies the compounding and formulation of the hydrophilic PP-based composition, but it is not excluded that the copolymer may also be a blend of individual copolymer materials falling under this definition.

The ethylene-propylene copolymer additive (or mixtures therefore if a blend is used) may be formulated in the range of from 5% to 40% by weight in the hydrophilic polypropylene-based composition used to make the synthetic fibers, in particular from 10% to 20% by weight of the hydrophilic polypropylene-based composition.

Nonlimiting examples of suitable commercially available ethylene-propylene copolymers are Affinity EG 8200G, Engage 8200, Infuse 9817, Vistamaxx 3000, Vistamaxx 6102, Vistamaxx 6202, Vistamaxx 6502, VERsify 4200, VERsify 4301.

The ethylene-propylene copolymer additive may comprise greater than 80 wt.% of polypropylene units with isotactic stereochemistry and random ethylene distribution. Examples of such copolymers are commercially available as the Vistamaxx series from ExxonMobil, for example Vistamaxx 6202, Vistamaxx 6502 and Vistamaxx 7050. These copolymers are primarily composed of isotactic propylene repeat units with random ethylene distribution, produced using a metallocene catalyst technology. They are sold as pellets which can be molten with the PP matrix pellets before spinning the synthetic fibers.

Without wishing to be bound by theory, the inventors believe that the presence of an ethylene-propylene copolymer additive as defined by the present invention can provide amorphous micro areas at the surface of the synthetic fibers that are believed to enhance the blooming and at the same time the anchoring of the surfactant at the surface of the synthetic fibers, and thus promote the durability of the hydrophilicity of the carded nonwoven made thereof.

The ethylene-propylene copolymer additives suitable in the present invention are relatively amorphous, which may be linked to a relatively low isotactic character of the propylene functional portions of the copolymers. Among the various commercially available ethylene-propylene copolymer additives, certain combination of molecular weight and enthalpy of crystallization are believed to be preferable.

As detailed in the example section below, the enthalpy of crystallization of the ethylene-propylene copolymer additive may typically be in the range of from about 0 J/g to about 40 J/g, in particular from 0.5 J/g to 40 J/g.

In addition, or alternatively, the peak molecular weight of the ethylene-propylene copolymer may be above 100,000 g/mol, preferably above 140,000 g/mol (as measured by the Peak Molecular Weight (Mp) Measurement Method described below), and up to 800,000 g/mol.

Table 1 below reports the measured values for the Melting Peak Temperature, Enthalpy of Crystallization and Molecular Weight of different Vistamaxx^{®} additives that may be used in the present invention.

**Table 1**

| Ethylene-propylene Copolymer Additive | Melting Peak Temperature (Tmp) | Crystallization Enthalpy [J/g] | Peak Molecular Weight [g/mol] |
|---|---|---|---|
| Preferred Range: | 40 - 100 °C | 0.5 - 40 | >100,000 |
| Vistamaxx 6202 | 62.5 °C | 7.8 | 146,746 |
| Vistamaxx 6102 | 62.3 °C | 8.7 | 310,656 |
| Vistamaxx 7020BF | 61.3 °C | 0.9 | 146,746 |
| Vistamaxx 7050 | 59.3 °C | 16.0 | 146,781 |
| Vistamaxx 8880 | 93.9 °C | 32.3 | 23,351 |

### Hydrophilic melt additive

The hydrophilic polypropylene-based composition comprises a hydrophilic additive. As for the other components indicated, the hydrophilic polypropylene-based composition may comprise a single, or a mix of two or more, of such hydrophilic additives.

Hydrophilic additives are substances that have an affinity for water and can be used to modify the surface properties of materials such as polypropylene. The hydrophilic additive is typically a surfactant. Surfactants are molecules that can reduce the surface tension between a liquid (such as water) and a solid (such as polypropylene), allowing the liquid to spread more easily over the surface. They can improve the wetting properties of polypropylene, making it more receptive to an aqueous fluid in particular urine.

Surfactants are amphiphilic molecules having a hydrophilic head and a hydrophobic tail. The hydrophilic head is oriented outwardly towards the surface of the fibers, thus providing for its hydrophilic character, while the hydrophobic tail remains in the polymer matrix. The synthetic fibers of the invention have particularly durable hydrophilic properties, enabled by the compatibility of the ethylene-propylene copolymer additive and the hydrophilic melt additive.

Ethoxylated surfactants are commonly used as hydrophilic additives for polypropylene and can be used in the present invention. These surfactants have a hydrophilic ethylene oxide chain attached to a hydrophobic alkyl chain. The hydrophilic portion allows for better wetting on polypropylene surfaces. An example of ethoxylated surfactant is HydroSorb^{®} 1012 from Goulston Technologies.

More generally nonionic surfactants, which do not carry an electrical charge in solution, are also suitable for polypropylene. They offer good compatibility and stability in the melt processing of polypropylene. Nonionic surfactants can help reduce the surface tension of polypropylene, enhancing its wetting and spreading properties.

Polymeric surfactants, also known as polymeric emulsifiers, are macromolecules that possess both hydrophilic and hydrophobic regions. These surfactants can provide excellent compatibility and stability in polypropylene systems and may also be used in the present invention.

Hydrophilic melt additives are typically provided as masterbatch in the form of pellets than can be incorporated by homogenous mixing in the molten polyolefin matrix. Commercial examples of hydrophilic melt additives particularly compatible with a propylene-based metallocene-catalyzed polyolefin are PPM 15560 from Techmer (hydrophilic PP masterbatch) and Brij S2 (Croda). Further, Brij S10 (from Croda,) Unithox 450, Unithox 720 and Unithox 750 (from Baker Hughes) can be used. PPM 15560 can for example be used in a dosage of 0.5 weight percent of the masterbatch, Brij S2 and Brij S10 in a dosage of preferably 2 weight percent of the active. Techsurf^{®} melt additives from Techmer have been used to impart hydrophilicity to polyolefin fibers, nonwoven fabrics, and specialty plastic applications, and are also useful in the present invention.

US6,146,757 discloses a hydrophilic melt additive comprising a blend of a first wetting agent and a second wetting agent. The first wetting agent is at least one water insoluble nonionic alkoxylated alkyl phenol, and the second wetting agent is at least one compound selected from the group consisting of an alkoxylated fatty alcohol and a water-soluble, nonionic, nonhydrolyzable polyoxyalkylene-modified organosilicone polymer. A particular example is the first wetting agent is an ethoxylated nonylphenol having about 4 moles of ethylene oxide and the second wetting agent is a water-soluble, nonionic, non-hydrolyzable polyoxyalkylene-modified organosilicone polymer. However, this example is not limiting the present invention, which can be reduced in practice with other melt additives, as exemplified above.

The additives of the Brij^{®} series from Croda are ethoxylated alcohols of the general formula: 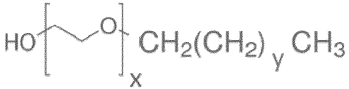 with x ranging from 2 to 100 and y ranging from 12 to 24, in particular y = 16 (stearyl).

For example, Brij S2 with x = 2 and y = 16 has a low molecular weight of 386 g/mol, which presumably facilitates the diffusion to the surface. These ethoxylated alcohols may be a more cost-effective alternative to the above-mentioned blend. The blends described in US6,146,757 were found to enable a stronger hydrophilic effect, while Brij S2 enables a milder hydrophilic effect. The additive may be thus selected depending on the application purpose.

### Fatty acid amides

The synthetic staple fibers may optionally comprise a fatty acid amide added to the PP-based matrix. This was found to increase the softness of the carded nonwoven obtained and may for example be relevant for applications that are in contact with the skin, such as the topsheet of an absorbent article.

Oleamide and erucamide are fatty acid amides commonly used as slip agent in polyethylene or polypropylene nonwovens. These compounds can be introduced as melt additives in the PP base as for the other additives. Oleamide is derived from mono-unsaturated C18 oleic acid, while erucamide is the amide of C22 mono-unsaturated erucic acid. The molecules of fatty acid amides are known to migrate at the surface of the fibers and thus provide a self-replenishing surface lubrication (effect referred to as "blooming"). This can reduce the coefficient of friction of the nonwovens made from these fibers or filaments. US3,454,519 (Hulse et al.) for example discloses improved textile fibers prepared from isotactic polypropylene resin containing from about 0.01% to about 1.0% by weight of erucamide. Oleamide has a lower basis weight and is known to migrate to the film surface more rapidly than erucamide. Stearamide is also known to reduce the coefficient of friction when used as melt additive to the polymer melt used to make the nonwoven's fibers. Blend of such fatty acids may also be used. Such a blend of stearamide and erucamide is for example disclosed in US6,740,609 (Peng et al.).

Typically, the heat generated during the making of the nonwoven and/or on an absorbent article's converting line is enough for the blooming to occur. The migration of the molecules of fatty acid amides may also be accelerated up by heating the nonwoven at 40-50°C for a few hours if necessary. Sufficient heat can also be provided during the thermo-bonding (calendering) step used in the consolidation of the nonwoven. Typically, the web is wound into rolls within a few seconds after the thermo-bonding step. Typically, under these conditions a temperature of around 40°C is maintained over several hours inside the rolls. It has been found that the insertion of an additional heating step, e.g., by using standard drying equipment (such as for drying surfactant coatings), may accelerate the blooming of erucamide and enables an overall reduction of the coefficient of friction. This can be used for further usage reduction of the fatty acid amide, e.g., erucamide. However, it requires an additional process operation which may not be available at the production line and its costs further energy. So, it needs to be decided in each individual case if to insert an additional heating step for further e.g., erucamide usage level reduction.

Any fatty acid amide melt additives that can reduce the coefficient of friction of the nonwoven may be used. Suitable fatty acid amides include those deriving from a mixture of C12-C28 fatty acids (saturated or unsaturated) and primary or secondary amines. A suitable example of a primary fatty acid amide includes those derived from a fatty acid and ammonia as illustrated in [1]. 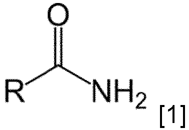 where R has a number of carbon atoms ranging from 11 to 27, in particular from 16 to 22 fatty acid. The fatty acid amide may be comprised of a single compound, e.g., erucamide, or a mixture thereof. The synthetic staple fibers may typically comprise at least 0.05%, preferably from about 0.1% to about 2%, of the fatty acid amide or mixture thereof, by weight of the nonwoven.

### Other optional components

Optional components may be further added to the polypropylene-based matrix as is known in the art, in particular pigments, plasticizer, UV stabilizer, antioxidant, brightener, colorant. Inorganic particles such as TiO₂ particles may be present in the synthetic staple fibers at level at least 0.25% by weight of the nonwoven, and up to 2%, for example 0.5% by weight of the nonwoven.

### Other synthetic fibers

The inventive staple fibers can also be blended with other synthetic fibers, e.g. with up to 10%, or 20%, or 40%, or 60%, or 80% by weight of the nonwoven of other synthetic fibers (for example bicomponent fibers such as PP/CoPP or PET/PE core/sheath bicomponent fibers, especially if the nonwoven is an air-through bonded nonwoven). Mixing other synthetic fibers also enables to dial in the degree of hydrophilicity of the nonwoven.

The nonwovens of the invention typically comprise at least about 10%, at least about 20%, at least about 30%, at least 40%, or at least 50% (all % by weight unless indicated otherwise) of synthetic staple fibers comprising or consisting of the hydrophilic polypropylene-based composition as described above.

In particular, the nonwoven may comprise from about 50% up to about 100% by weight of the synthetic staple fibers comprising or consisting of the hydrophilic polypropylene-based composition for applications where strong hydrophilic durability is desired, such as in topsheet or acquisition layer for absorbent article.

Natural or other synthetic staple fibers can be added to the inventive staple fibers mix. Other synthetic fibers, such a mono-component PE or mono-component PP fibers or mono-component PET fibers, or bicomponent fibers such as PP/PE core/sheath bicomponent fibers or PET/PE core/sheath bicomponent fibers or PET/CoPET core/sheath bicomponent fibers may be comprised in the synthetic staple fibers.

Adjusting the amount of durably hydrophilic fibers of the invention in a blend with other synthetic fibers allows for control over the degree of hydrophilicity, particularly durable hydrophilicity, in the resulting nonwoven. In certain applications for absorbent articles, it may be desired for the nonwoven to partially lose its original hydrophilicity during use, while maintaining some level of hydrophilicity.

The staple fibers may comprise a mix of durably hydrophilic fibers and temporary hydrophilic fibers. Conventional synthetic fibers can be for example rendered non-durably hydrophilic using a non-durable spin finish as is known in the art. Inventive fibers (from about 10% to about 90% by weight of the nonwoven) that are durably hydrophilic can be blended with the non-durably hydrophilic synthetic fibers to provide the nonwoven with partially durable hydrophilicity after exposure to body liquids. This controlled loss of hydrophilicity can be advantageous for creating a barrier layer that prevents rewet, i.e. liquid from traveling back towards the wearer's skin after the initial gush of body liquids. The durably hydrophilic fibers ensure that the nonwoven remains capable of accepting and allowing subsequent gushes of body liquid to pass through. An adjusted degree of durable hydrophilicity in the nonwoven is particularly useful in applications such as an acquisition layer. It is expected that the inventive durably hydrophilic fibers may be present in a weight amount of from 10% to 50%, in particular from 10% to 20% of the carded nonwoven for such type of applications. This can enable an acquisition layer with evolving barrier properties in use (majority of fibers getting hydrophobic), but still a defined basic level of local hydrophilicity, enabling continued water permeability.

The acquisition layer can be typically resin bonded or air-through bonded. In air-through bonding, the inventive fibers can be bonded to other synthetic fibers with a similar melting point or simply blended to the other fibers (in that latter case typically up to a maximum of 20% of durably hydrophilic fibers by weight of the nonwoven).

Air-through bonded nonwovens require bi-component fibers, which may be inventive durably hydrophilic fibers as described above, or conventional bico fibers, or a mixture of both type of fibers. When different bico fibers are used, the difference in melting points of their sheath may be preferably less or equal than about 20°C. This enables the creation of air-through bonded nonwovens using inventive PP/CoPP or PET/CoPP core sheath bicomponent fibers alongside standard synthetic PP/PE or PET/PE core sheath bicomponent fibers. For example, bicomponent fibers having PE sheath with a melting point of approximately 130 °C are compatible with bicomponent fibers having a durably hydrophilic CoPP sheath with a melting point of around 140 °C.

The inventive fibers can be used for making resin bonded acquisition layers with 100% or dialed-in durable hydrophilicity: about 20% durably hydrophilic PET/PP bico or PP mono fibers can be admixed to conventional PET mono fibers, which enables to dial-in (durable) hydrophilicity into a convention acquisition layer, which is today only available at the junction points (via a durably hydrophilic resin binder). Alternatively, a 100% PP based resin bonded acquisition layer (PET/PP bico or PP mono) can be made, which is 100% durably hydrophilic.

### Natural fibers

The synthetic staple fibers of the invention may be mixed with natural fibers if desired to make a carded nonwoven comprising a mix of synthetic and natural fibers. Natural fibers may be selected from the group consisting of wheat straw fibers, rice straw fibers, flax fibers, bamboo fibers, cotton fibers, jute fibers, hemp fibers, sisal fibers, bagasse fibers, hesperaloe fibers, miscanthus, marine or freshwater algae/seaweeds and combinations thereof. The natural fibers may for example be selected from the group consisting of cotton fibers, silk fibers, bamboo fibers, or mixtures thereof. The natural fibers may in particular comprise or consist of cotton fibers. Cotton fibers are natural cellulosic fibers that have good liquid acquisition, and good breathability. Incorporating cotton fibers or similar soft natural fibers renders the nonwoven more environmentally friendly as these are renewable resources while improving the fluid handling properties of the nonwoven. This is particularly desirable when using the nonwoven as a topsheet. A calendered nonwoven of the invention may typically comprise from 5% to 20% of natural fibers, by weight of the nonwoven, for example 15% by weight of the nonwoven.

### Examples of Carded Staple Fibers

Durably hydrophilic staple fibers according to the invention were produced using a PP-based polymer matrix (PP-H or CoPP), an ethylene-propylene copolymer additive and a hydrophilic melt additive. These components were melted to make a hydrophilic polypropylene-based composition and staple synthetic fibers were formed therefrom. The synthetic fibers were either monocomponent fibers or alternatively bicomponent fibers of the sheath/core type with the sheath component being the hydrophilic polypropylene-based composition. Comparative staple fibers without the ethylene-propylene copolymer additive were produced under the same conditions.

The staple fibers were carded and then submitted to the Sink Time Test. The Sink Time Test, as detailed below, can be used to measure the durability of the hydrophilic treatment of the carded nonwoven. The Sink Time Values at the second cycle measures the hydrophilicity of the nonwoven after a first wash-off (the shorter the time, the higher the hydrophilicity). The compositions of the inventive and comparative fibers and their respective Sink Time at the second cycle are reported in Tables 2a (mono fibers) and 2b (bicomponent fibers) below:

**Table 2a**

| **Ex. Nr.** | **PP Base** | **Type of Fibers** | **Hydrophilic Melt Additive** | | **Ethylene-Propylene Copolymer** | | **Sink Time of the 2nd cycle (max = 2h)** |
|---|---|---|---|---|---|---|---|
| | | | **Name** | **Wt. %** | **Name** | **Wt. %** | |
| 1 (reference) | PP-H | Mono | - | 0 | - | - | 2 h |
| 2 (comparative) | PP-H | Mono | Techmer PPM 15560 | 3 | - | - | 2 h |
| 3 (comparative) | PP-H | Mono | Hydrosorb 1012 | 10 | - | - | 2 h |
| 4 (inventive) | PP-H | Mono | Techmer PPM 15560 | 3 | Vistamaxx 7050BF | 15 | 3 s |
| 5 (inventive) | PP-H | Mono | Hydrosorb 1012 | 10 | Vistamaxx 7050BF | 15 | 16 s |
| 6 (inventive) | PP-H | Mono | Techmer PPM 15560 | 3 | Vistamaxx 7020BF | 15 | 4 s |
| 7 (inventive) | PP-H | Mono | Techmer PPM 15560 | 3 | Vistamaxx 8880 | 15 | 4 s |

As can be seen from the results reported in Table 2a, the synthetic staple mono fibers according to the invention displayed excellent hydrophilic durability properties compared to the non-inventive synthetic staple fibers.

**Table 2b**

| **Ex. Nr.** | **PP Base** | **Type of Fibers** | **Hydrophilic Melt Additive** | | **Ethylene-Propylene Copolymer** | | **Sink Time of the 2nd cycle (max = 2h)** |
|---|---|---|---|---|---|---|---|
| | | | **Name** | **Wt. %** | **Name** | **Wt. %** | |
| 8 (reference) | coPP | Bico (coPP/PP) | - | 0 | - | - | 2 h |
| 9 (comparative) | coPP | Bico (coPP/PP) | Techmer PPM 15560 (sheath) | 3 | - | - | 8 min |
| 10 (inventive) | coPP | Bico (coPP/PP) | Techmer PPM 15560 (sheath) | 3 | Vistamaxx 7050BF | 15 | 7 s |
| 11 (inventive) | PP-H | Bico (PP/PET) | Techmer PPM 15560 (sheath) | 3 | Vistamaxx 7050BF | 15 | 3s |

As can be seen from the results reported in Table 2b, the same conclusion applies to synthetic staple bi-component fibers, wherein the sheath of the bico fibers is formed from a hydrophilic-based composition according to the invention.

As can be seen from the data in table 2, the inventive carded nonwoven examples which comprised an ethylene-propylene copolymer additive and a hydrophilic melt additive fibers had a much lower Sink Time at the second cycle than the comparative stable fibers, showing that the synthetic fibers of the invention are still hydrophilic after a first simulated gush (2nd cycle value).

The inventive examples were subjected to further cycles of the Sink Time Test. For all inventive examples, the Sink Time was maintained below 20 s (which is considered as highly hydrophilic) for a total of at least 5 cycles.

### Consolidated carded nonwovens

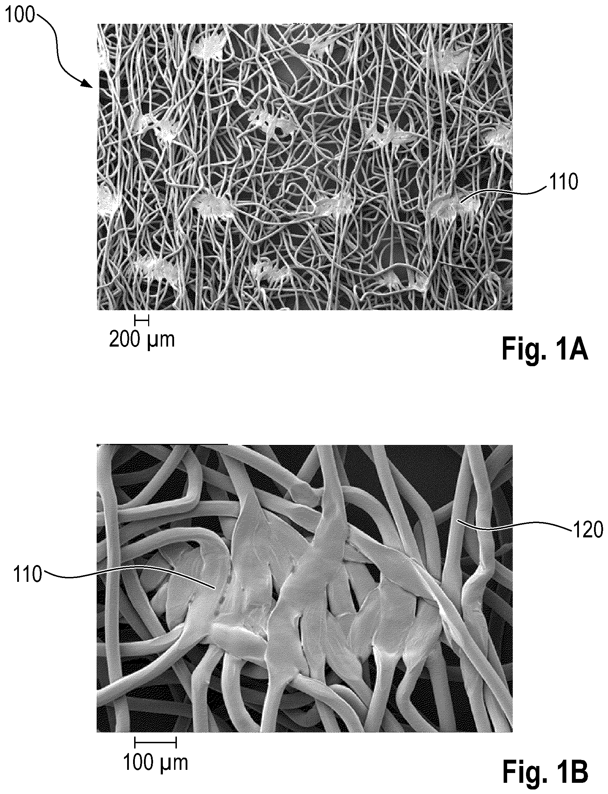

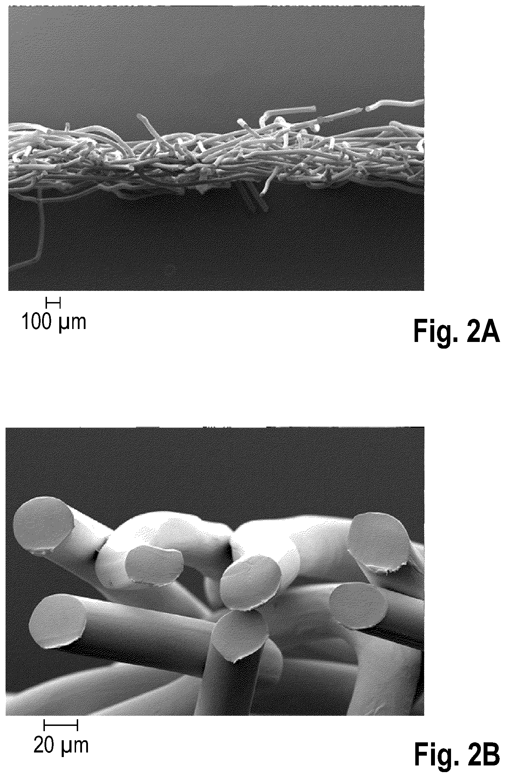

The carded staple fibers of example 3 were consolidated by calendering. SEM pictures of the resulting consolidated nonwoven at different magnification of the calendered (top) surface of the nonwoven 100 are shown in Fig. 1A-1B, and cross-section view are shown in Fig. 2A,B. The calendering pattern consisted of oval bond points 110. The individual staple synthetic fibers 120 were all durably hydrophilic fibers according to the invention.

### Absorbent article

As used herein, "absorbent articles" refers to personal hygiene devices that are placed in the crotch of a wearer to absorb and contain body exudates. Absorbent articles include baby care articles, feminine care articles or adult incontinence articles.

Baby care articles are products intended for babies, toddlers and/or children, relating to disposable absorbent articles including taped diapers, pant diapers, absorbent inserts, infant and/or toddler care wipes, and infant and/or toddler bibs.

Feminine care articles are products relating to catamenial pads, incontinence pads, interlabial pads, panty liners, pessaries, sanitary napkins, tampons and tampon applicators, and/or wipes.

Adult incontinence articles are products intended for adults, relating to disposable absorbent articles including diapers, pants, absorbent inserts, incontinence pads, panty liners, and vaginal inserts.

Absorbent articles are typically disposable and are preferably recyclable.

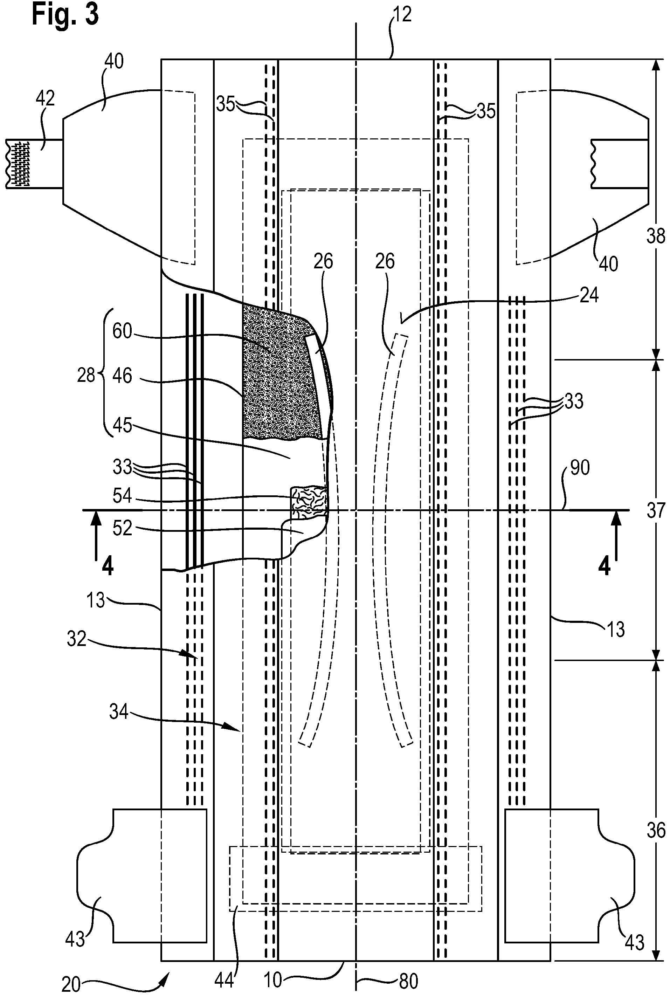

As used herein, "diapers" refers to absorbent articles generally worn by babies, infants and incontinent adults about the lower torso so as to encircle the waist and legs of the wearer and that is specifically adapted to receive and contain urinary and fecal waste. Diapers are typically proposed as taped diapers or pant diapers. Taped diapers have a fastening system (as illustrated in Fig. 3 for example), where the waist opening and leg openings are formed when the diaper is applied onto the wearer by releasably attaching the longitudinal edges of the front waist region and back waist region to each other. In pant diapers, on the other hand the longitudinal edges of the waist regions are attached to each other to form a pre-formed waist opening and leg openings. A pant diaper is placed in position on the wearer by inserting the wearer's legs into the leg openings and sliding the pant diaper into position about the wearer's lower torso. A pant may be pre-formed by any suitable techniques including, but not limited to, joining together portions of the absorbent article using re-fastenable and/or non-refastenable bonds (e.g., seam, weld, adhesive, cohesive bond, fastener, etc.). A pant may be pre-formed anywhere along the circumference of the article (e.g., side fastened, front waist fastened).

An exemplary, non-limiting absorbent article according to the invention is discussed herein in relation to Figures 3-4. Figure 3 is a plan view of such an exemplary diaper 20, in a flat-out state, with portions of the diaper being cut-away showing the different layers of the diaper. This diaper 20 is shown for illustration purpose only, as the nonwoven of the present invention may be comprised in a wide variety of diapers or other absorbent articles, such as pant diapers having pre-formed side seams. The side seams of pant articles can be opened by cutting or otherwise, if it is desired to place the pant in a flattened-out configuration similar to Fig. 3.

As illustrated in Figures 3-4, the absorbent article comprises a topsheet 24 on its wearer-facing side, a backsheet 25 on its garment-facing side, and an absorbent core 28 between the topsheet 24 and the backsheet 25. The absorbent core 28 comprises at least one absorbent layer 60, typically comprising superabsorbent polymer particles ("SAP") and optionally cellulose fibers, and a core wrap. The absorbent layer is disposed between a top core wrap layer 45 and a bottom core wrap layer 46.

The SAP layer typically has a pre-determined outline (periphery) as considered in the plane formed by the article when flattened out. This outline may be substantially rectangular or have another shape such as a sand-hour or dog-bone shape. The absorbent core 28 may further comprise at least one longitudinally-oriented channel-forming area 26, which is an area substantially free of absorbent material defined within the absorbent layer. The channel-forming area(s) 26 facilitate(s) the distribution of a fluid along the length of the absorbent article.

The absorbent article 20 further comprises an acquisition layer 52 between the topsheet 24 and the absorbent core 28. The article may also comprise an acquisition-distribution system comprising the acquisition layer 52 in combination with a distribution layer 54 between the acquisition layer and the absorbent core. The absorbent article, especially when the absorbent layer comprises cellulose fibers, may comprise a single layer as acquisition-distribution layer, referred herein as acquisition layer, between the topsheet and the absorbent core.

Typical example of acquisition layer 52, which may be used alone or with a distribution layer, is a surfactant treated, latex bonded, nonwoven acquisition layer. The distribution layer 54 may be a layer of cross-linked cellulose fibers, especially when the absorbent core 28 is an airfelt-free absorbent core. Another example of acquisition layer 52 or distribution layer 54 that can be used in the present invention is a spunlace layer.

The durably hydrophilic, carded nonwoven of the present invention may be used in any layers of the absorbent article where a durably hydrophilic properties is desired. The carded nonwoven of the present invention may thus especially be one of a topsheet, an acquisition layer or a top layer core wrap in an article of the invention such as a diaper.

As illustrated in Fig. 3, the absorbent article, whether a taped diaper or a pant diaper, can be notionally divided in a front waist region 36, a back waist region 38 opposed to the first waist region 36, and a crotch region 37 located between the front waist region 36 and the back waist region 38. The crotch region, the front waist region and the back waist region are hereby defined as delimiting each one third of the length of the absorbent article along the longitudinal centerline 80. The longitudinal centerline 80 is the imaginary line separating the diaper along its length in two equal right and left halves. The transversal centerline 90 is the imaginary line perpendicular to the longitudinal centerline 80 in the plane of the flattened-out diaper and going through the middle of the length of the diaper. The periphery of the diaper 20 is defined by the outer edges of the diaper. The longitudinal edges 13 of the diaper may run generally parallel to the longitudinal centerline 80 of the diaper 20 and the front waist edge 10 and the back waist edge 12 typically run generally parallel to the transversal centerline 90 of the diaper 20. However, these article edges do not need to be straight, at they may be curved to better fit the wearer.

Further, the absorbent article may comprise other optional but conventional elements, which are not represented for simplicity, such as a back waist elastic feature, a front waist elastic feature, a lotion applied onto the body-facing surface of the topsheet, or a urine indicator disposed on the inner side of the backsheet that changes color when contacted with urine.

The topsheet 24, the backsheet 25, and the absorbent core 28 may be assembled in a variety of well-known configurations, such as by gluing, heat embossing, ultrasonic bonding or combinations thereof. Exemplary diaper configurations are described generally in US3,860,003; US5,221,274; US5,554,145; US5,569,234; US5,580,411; and US6,004,306.

The topsheet 24 forms the wearer-facing surface of the article that is in intimate contact with the skin of the wearer, in particular the perineal area. At least a portion of and typically all of the topsheet is liquid permeable, permitting liquid bodily exudates to readily penetrate through its thickness. The topsheet may consist or comprise a carded nonwoven according to the invention, benefitting from the excellent hydrophilicity of the nonwoven of the invention, especially when the staple fibers comprise a combination of natural and synthetic fibers.

The topsheet may have one or more layers. The topsheet may be apertured or non-apertured, and may have any suitable three-dimensional features, and/or may have a plurality of embossments (e.g., a bond pattern). Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet.

The backsheet 25 is generally the portion of the absorbent article 20 that constitutes all or a part of the garment-facing surface of the absorbent article. The backsheet 25 may be joined at least partially to the topsheet 24, the absorbent core 28, or other layers of the article by any attachment methods known to those of skill in the art. The backsheet prevents, or at least inhibits, the bodily exudates absorbed and contained in the absorbent core from soiling articles such as bedsheets, undergarments, and/or clothing. The backsheet is typically liquid impermeable, or at least substantially liquid impermeable.

The backsheet typically comprises a thin impermeable plastic film, usually a thermoplastic film having a thickness of about 0.01 mm to about 0.05 mm. The backsheet material may be breathable, which permit vapors to escape from the absorbent article, while still preventing, or at least inhibiting, bodily exudates from passing through the backsheet. A breathable backsheet may have a Water Vapor Transmission Rate (WVTR) of from 1,000 to 15,000 g/m²/24h, or from 1,000 to 10,000 g/m²/24h, or from 1,500 to 10,000 g/m²/24h as measured using a PERMATRAN-W Model 101K (available from Mocon, Inc., Minneapolis, MN) or equivalent, according to Nonwovens Standard Procedure NWSP 70.4.R0(15) with the following specifications: experiments were carried out in a lab controlled at 23 °C ± 2 C° and 50 %RH ± 2 %RH and the instrument cells heated to 37.8°C (100°F).

The backsheet 25 may also comprise a backsheet outer cover nonwoven (not represented separately in the Figures). The backsheet outer cover nonwoven is typically a thin nonwoven material that is joined to the outer surface of the backsheet film. The outer cover nonwoven may thus form the garment-facing surface of the backsheet. The backsheet outer cover nonwoven may also comprise a bond pattern, apertures, and/or three-dimensional features, and may improve the feel of the backsheet.

The absorbent article 20 may also comprise inner barrier leg cuffs 34 and outer leg cuffs 32, as is known in the art. The inner barrier cuffs 34 can extend upwards from the surface of the article to provide retention of the waste, while the outer cuffs are typically formed in the plane of the chassis of the article as defined by topsheet and backsheet. These cuffs are preferably elasticized, as is known in the art, for example using elastic threads 33, 35 as represented in the Figures 3-4. While not illustrated, the absorbent article may also be in the form of a pant diaper comprising an inner and outer belt nonwovens as is known in the art, in which case the nonwoven of the invention may also be comprised in any of the inner and outer belt nonwovens. Pant diapers may also comprise a nonwoven according to the invention as part of the topsheet, barrier leg cuffs, or backsheet outer nonwoven cover of the pant diaper.

Moreover, the absorbent article may comprise a fastening system, such as an adhesive fastening system or a hook and loop fastening member, which can comprise tape tabs 42 disposed on back ears 40, such as adhesive tape tabs or tape tabs comprising hook elements, cooperating with a landing zone 44 (e.g. a nonwoven web providing loops in a hook and loop fastening system). While taped diapers typically comprise back ears 40, and front ears 43, these are typically not present in pant-type absorbent articles having pre-formed side seams.

The front and/or back ears may be separate components attached to the absorbent article or may instead be continuous with portions of the topsheet and/or backsheet such that these portions form all or a part of the front and/or back ears 40, 43. Also combinations of the aforementioned are possible, such that the front ears 43 and/or back ears 40 are formed by portions of the topsheet and/or backsheet while additional materials are attached to form the overall front and/or back ears 40, 43. The front and/or back ears may be elastic or non-elastic. Also, the front ears 40 may be applied as separate components attached to the absorbent article while the back ears (or parts thereof) may be continuous with portions of the backsheet and/or topsheet - or vice versa.

Absorbent articles such as diaper typically comprise an absorbent core between the topsheet and the backsheet. The absorbent core 28 may comprise an absorbent layer 60 and a core wrap 45, 46. The absorbent layer typically comprises superabsorbent polymer particles (SAP) optionally mixed with cellulose fibers. SAP are water-insoluble, water-swellable polymers capable of absorbing large quantities of fluids, as is known in the art. The term "superabsorbent polymer" refers herein to absorbent materials, typically cross-linked polymeric materials, which can absorb at least 10 times their weight of an aqueous 0.9% saline solution as measured using the Centrifuge Retention Capacity (CRC) test as indicated in EDANA method NWSP 241.0.R2 (19). The SAP may in particular have a CRC value of more than 20 g/g, or more than 24 g/g, or of from 20 g/g to 50 g/g, or from 20 g/g to 40 g/g, or 24 g/g to 35 g/g.

The absorbent layer 60 is typically sandwiched, and preferably immobilized, within a core wrap comprising a core wrap top layer 45 and core wrap bottom layer 46, so that the absorbent core can be easily integrated with the rest of the chassis of the absorbent article in a converting line. However some absorbent material exist that do not require a core wrap, such as an airlaid absorbent core or a coform absorbent layer.

Superabsorbent polymer particles are often mixed with cellulose fibers (airfelt cores). The absorbent core may alternatively comprise at least one layer of SAP, wherein the SAP particles are not mixed with cellulose fibers. The resulting layer of absorbent material may thus have a reduced thickness in the dry state, compared to conventional airfelt-based absorbent cores. The reduced thickness helps to improve the fit and comfort of the absorbent article for the wearer. The absorbent material may be free of cellulose fibers (however the absorbent core may still comprise some cellulose fibers in a nonwoven or tissue layer, such as a spunlace layer, for example as a core wrap layer).

Various absorbent core designs comprising a layer of SAP free of cellulose fibers have been proposed in the past, see for example in US5,599,335 (Goldman), EP1,447,066 (Busam), WO95/11652 (Tanzer), US2008/0312622A1 (Hundorf), WO2012/052172 (Van Malderen). In particular, the SAP printing technology as disclosed in US2006/024433 (Blessing), US2008/0312617 and US2010/0051166A1 (both to Hundorf et al.) may be used.

The layer of SAP may be typically deposited on at least one layer of the absorbent core wrap serving as substrate, such as the bottom core wrap layer or top core wrap layer. In the SAP-printing process as described in US2008/312,622A1 (Hundorf), a continuous layer of SAP is obtained by depositing SAP on each of the core wrap layers in a pattern having absorbent material land areas separated by absorbent material-free junction areas. The absorbent material land areas of the first layer correspond substantially to the absorbent material-free junction areas of the second layer and vice versa, so that a continuous layer of SAP is obtained when the two discontinuous layers are combined.

The absorbent core may comprise one or more glue layers, in particular an auxiliary glue may be disposed between the internal surface of at least one of the top and bottom core wrap layers and the absorbent layer to adhesively immobilize the absorbent material within the core wrap. A micro-fibrous thermoplastic adhesive net may also be used in airfelt-free cores as described in the above Hundorf references to immobilize SAP particles on at least one of the substrates formed by the top and bottom core wrap layers. These adhesives are not represented in the Figures for simplicity.

Other core constructions, for example comprising a high loft nonwoven substrate such as a carded nonwoven layer, having a porous structure into which SAP particles have been deposited, may also be used in the present disclosure.

The absorbent material layer may be present as a continuous layer on one of the core wrap layers. The absorbent layer may also be present discontinuously, for example, as individual pockets or stripes of absorbent material enclosed within the core wrap and separated from each other by material-free junction areas. The absorbent layer may also comprise one or more main continuous area and several pocket areas, in particular in the back of the absorbent core.

The basis weight (amount deposited per unit of surface) of the superabsorbent material may also be varied to create a profiled distribution of superabsorbent material, in particular in the longitudinal direction to provide more absorbency in the crotch portion of the article, but also in the transversal direction, or both directions of the core.

The core wrap is formed by one or two substrate layers that sandwiches the absorbent layer. The core wrap top layer 45 and the core wrap bottom layer 46 are also referred to in the art as core cover and dusting layer respectively. These core wrap layers are typically low basis weight nonwoven (typically less than 20 gsm, in particular from 8 gsm to 14 gsm), and may be in particular SMS nonwoven (Spunbond-Meltblown-Spunbond laminate), as is known in the art. The upper and lower core wrap layers may be any material capable of containing and providing a support for the absorbent material.

The core wrap layers may be made of the same or different materials, i.e. two nonwoven webs which have the same of different properties. The core wrap may also be made of a single, continuous nonwoven web, which is wrapped around the layer of absorbent material as this may simplify construction and comprising a single longitudinal seal, in this case the top core wrap and the bottom core wrap layers are made of the same web material.

The durably hydrophilic carded nonwoven of the present invention may be advantageously used as the core wrap top layer to facilitate the acquisition of the fluid into the absorbent layer.

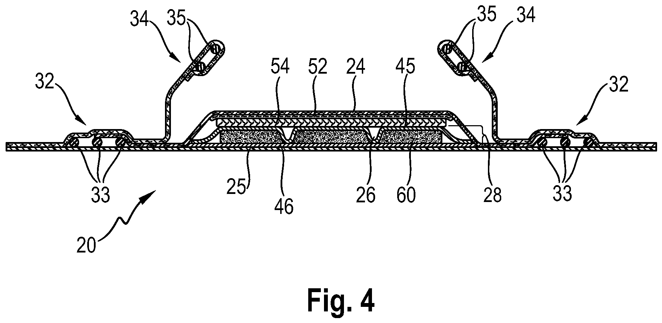

The core wrap layers may be bonded face to face, at least longitudinally as represented in Fig. 4, but other bonding configurations are possible, in particular a C-wrap configuration where one of the top or bottom core wrap layer is larger than the other, so that flaps can be folded around the absorbent material and attached to the other core wrap layer.

The absorbent core 28 optionally comprises at least one channel-forming area 26, where substantially no absorbent material is present (possibly some superabsorbent particles may be deposited accidental during core-making). The channel-forming area preferably does not extend to any of the side of the absorbent layer, and thus is completely surrounded by the absorbent material. The channel-forming area is typically elongated in the longitudinal direction, having a longitudinal length of from 20% and 80%, or from 20% to 70%, or from 30% to 60%, of the longitudinal length of the layer of SAP 60 (longitudinal length means the length as measured projected on the longitudinal axis). The absorbent core may typically comprise a pair of channel-forming areas disposed symmetrically on each side of the longitudinal axis 80, wherein these channel-forming areas may be straight, curved, or combinations thereof. Such a pair of channel-forming areas may be disconnected, as illustrated in Fig. 3. The channel-forming areas may also be connected, for example at one or both their extremities to form a U or O shape. Examples of channel-forming areas are disclosed in further details in WO2012170778A1, WO2012170781 (Kreuzer et al.).

The core wrap top layer 45 and core wrap bottom layer 46 are preferably bonded to each other through at least a portion of the length of the channel-forming area(s). This bond provides for structural integrity of the channels in dry and wet state. Any known bonding techniques known in the art may be used to provide for this bond, in particular one selected from adhesive bonding, thermo bonding, mechanical bonding, ultrasonic bonding, or any combinations thereof. An adhesive may be for example applied in the areas of the channels on the inner side of the top side and/or the inner side of the bottom side of the core wrap, typically by slot glue application or any other means, followed by the application of pressure in the areas of the channels to provide a good adhesive bonding in these areas. Exemplary patent disclosures of such adhesive bonding processes can be found for an airfelt or airfelt-free absorbent cores in WO2012/170798Al (Jackels et al.), EP2,905,000 (Jackels et al.) and EP2,905,001 (Armstrong-Ostle et al.).

Other bonding such as thermo bonding, mechanical bonding, ultrasonic bonding can also be used as additional bonding or as an alternative bonding. For example, an adhesive bonding may be reinforced by a thermo bonding, mechanical bonding or ultra-sonic bonding. Such thermo, mechanical or ultrasonic bonding can be applied on the channels through the external sides of the core wrap layers.

Typically, the channel bonds may generally have the same outline and shape as the channel-forming areas 26 in which they are contained but may be slightly smaller to allow for a safety margin (e.g. by a few mm) as some deviations from the optimal registration may happen during high speed process. The channel-forming area(s) form three-dimensional channel(s) during use as the rest of the absorbent layer absorbent core absorbs a fluid and swells. The channel-forming area(s) are optional in the present invention.

The total amount of SAP present in the absorbent core is adapted to the need of the expected wearer of the article. Diapers for newborns require less SAP than infant or adult heavy incontinence diapers. The amount of SAP in the core may be for example comprised from about 2 g to 50 g, in particular from 5 g to 40 g for typical enfant diapers. The average SAP basis weight within the absorbent core may be for example of at least 50, 100, 200, 300, 400, 500 g/m² or more, or from 200 g/m² to 400 g/m². The average SAP basis weight is the total amount of SAP in the core divided by the area delimited by the periphery of the SAP layer (including any channel-forming areas if present).

### Test methods

### Sink Time Test

The Sink Time Test can be used to measure the durability of the hydrophilic properties of carded staple fibers (before or after consolidation). Approx. 5 g of the carded fibers are put into a standardized basket as described in DIN EN ISO 9073-6. Then this basket is placed on the surface of distilled water in a beaker. The time between placing the basket on the surface and the moment the basket has sunk completely below the surface is measured. The measurement is stopped at 2 hours if the basket did not sink until that time, and the value 2 hours is reported. The measurement is repeated several times (in several "cycles"). Between the cycles the fibers (or the nonwoven sample) are thoroughly dried at room temperature.

The Sink Time Test for the second cycle is used to assess the durability of the hydrophilicity of the fibers, as hydrophilic process aids in fiber manufacturing (spin finish) mostly wash off during the first cycle. The Sink Time of the second cycle is indicative of the degree of hydrophilicity of the fiber itself and is a more conservative measure of the fiber hydrophilicity.

### Peak Molecular Weight (Mp) Measurement Method

The peak molecular weight is determined using a gel permeation chromatography (GPC) method. GPC is a well-known method wherein polymers are separated according to molecular size, the largest molecule eluting first. The peak molecular weights referred to herein can be determined with gel permeation chromatography (GPC) using polystyrene calibration standards, according to ASTM D5296. The molecular weight of any polymer or unknown polymer measured using GPC so calibrated is the styrene equivalent molecular weight, which herein is defined as the "peak molecular weight". Suitable solvents and temperatures are employed with GPC in order to achieve adequate molecular weight separation and resolution.

### Melting Peak Temperature and Enthalpy of Crystallization Test Method

The Melting Peak Temperature (Tmp) and Enthalpy of Crystallization of a polymer is determined using ASTM D3418-15 ("Standard Test Method For Transition Temperatures And Enthalpies Of Fusion And Crystallization Of Polymers By Differential Scanning Calorimetry") with the following additional guidance. Dry nitrogen is used as the purge gas in the differential scanning calorimeter (DSC). The rate of increase of temperature in the DSC is 10 °C/min, and the rate of decrease of temperature in the DSC is 1 °C/min. The mass-normalized enthalpy of crystallization is calculated as specified in section 11.4 based on the curve corresponding to decreasing temperature (at 1° C./min) and is reported as the "Enthalpy of Crystallization" in units of joules per gram (J/g) to the nearest 0.1 J/g. The Melting Peak Temperature (Tmp) is measured as indicated in section 10.1 of ASTM D3418-15.

### Misc

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

Unless indicated otherwise, the description and claims refer to the absorbent core and article before use (i.e. dry, and not loaded with a fluid) and conditioned at least 24 hours at 21 °C +/- 2 °C and 50 +/- 5% Relative Humidity (RH).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. An absorbent article (20) comprising a carded nonwoven (100), the carded nonwoven comprising synthetic staple fibers (110), wherein the synthetic staple fibers comprise or consist of a hydrophilic polypropylene-based composition, said hydrophilic polypropylene-based composition comprising a polypropylene-based matrix, an ethylene-propylene copolymer additive and a hydrophilic melt additive.

2. The absorbent article according to claim 1, wherein the polypropylene-based matrix is a propylene homopolymer (h-PP), a propylene co-polymer (CoPP) or a mixture thereof.

3. The absorbent article according to any of the preceding claims, wherein the synthetic staple fibers are either a) mono-component fibers consisting of the hydrophilic polypropylene-based composition, or b) bi-component fibers comprising a sheath/core structure wherein the sheath consists of the hydrophilic polypropylene-based composition, or a mixture of fibers according to a) and b).

4. The absorbent article according to any of the preceding claims, wherein the ethylene-propylene copolymer additive is metallocene catalyzed and has a peak molecular weight between 100,000 g/mol and 800,000 g/mol, preferably between 140,000 g/mol and 400,000 /mol, wherein the peak molecular weight is measured by the Peak Molecular Weight Measurement Method indicated herein.

5. The absorbent article according to any of the preceding claims, wherein the hydrophilic melt additive comprises or consist of one or more ethoxylated surfactant, in particular wherein one or more ethoxylated surfactants having the formula: 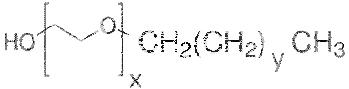 with x ranging from 2 to 100 and y ranging from 10 to 24.

6. The absorbent article according to any of the preceding claims, wherein the hydrophilic melt additive comprises a first wetting agent and a second wetting agent, wherein the first wetting agent is at least one water-insoluble, nonionic alkoxylated alkyl phenol, and wherein the second wetting agent is at least one compound selected from the group consisting of an alkoxylated fatty alcohol and a water-soluble, nonionic, non-hydrolyzable polyoxyalkylene-modified organosilicone polymer.

7. The absorbent article according to any of the preceding claims, wherein the hydrophilic polypropylene-based composition comprises by weight of the hydrophilic polypropylene-based composition:
- at least 50%, preferably at least 65%, of the polypropylene-based matrix;
- from 5% to 25%, preferably from 10% to 20%, of the ethylene-propylene copolymer additive.

8. The absorbent article according to any of the preceding claims, wherein the carded nonwoven is one of:
- a carded calendared nonwoven, or
- an air-through bonded carded nonwoven, or
- a resin bonded carded nonwoven, or
- a needle-punched carded nonwoven.

9. The absorbent article according to any of the preceding claims, wherein the carded nonwoven has a basis weight in the range of from about 10 g/m² to about 40 g/m².

10. The absorbent article according to any of the preceding claims, wherein the carded nonwoven is an air-through bonded carded nonwoven comprising a mixture of:
- bi-component fibers with PE sheath; and
- bi-component fibers with CoPP sheath;
wherein the CoPP sheath consists of the hydrophilic polypropylene-based composition.

11. The absorbent article according to any of the preceding claims, wherein the carded nonwoven is either:
- a resin bonded carded nonwoven comprising a mix of PET fibers and PP fibers, wherein the PP fibers comprise the hydrophilic polypropylene-based composition; or
- a resin bonded carded nonwoven comprising PET fibers and PET/PP bicomponent fibers, wherein the PP sheath of the bicomponent fibers consists of the hydrophilic polypropylene-based composition.

12. The absorbent article according to any of the preceding claims, wherein the polypropylene-based matrix has a Melting Peak Temperature (Tmp) in the range of about 130 °C to about 180 °C and the ethylene-propylene copolymer additive has a Melting Peak Temperature (Tmp) in the range of about 40 °C to about 100 °C, wherein the Melting Peak Temperature is measured by the Melting Peak Temperature Test Method described herein.

13. The absorbent article according to any of the preceding claims, wherein the carded nonwoven has a Sink Time of less than 10 min at the Second Cycle, preferably of less than 1 min, more preferably less than 30 s, as measured according to Sink Time Method disclosed herein.

14. The absorbent article according to any of the preceding claims, wherein the enthalpy of crystallization of the ethylene-propylene copolymer additive is in the range of about 0.5 J/g to about 40 J/g as measured by the Enthalpy of Crystallization Test Method described therein.

15. The absorbent article according to any of the preceding claims, wherein the absorbent article comprises a topsheet (24), an acquisition layer (52), an absorbent core (28) and a backsheet (25), and the carded nonwoven is comprised in at least one of the topsheet, the acquisition layer or the absorbent core.
